# EUROPEAN PATENT APPLICATION

(11) **EP 2 116 884 A1**
(43) Date of publication of application: **11.11.2009**
(21) Application number: 09005054.3
(22) Date of filing: 06.04.2009
(51) Int. Cl.: G02B 21/36, A61B 1/04, H04N 1/00

(54) **Low cost production, use and transmission of data in image microscopy**

(30) Priority: 10.04.2008 IT PA20080009
(71) Applicant: Bellina, Livia, 90144 Palermo (IT)
(72) Inventor: Bellina, Livia, 90144 Palermo (IT)

(57) **Abstract**

Until now the use for teaching purposes or remote transmission of microscopical images was bound to sophisticated and expensive devices.
Using a simple cellphone camera it is possible to take pictures from any microscope and send them by mms to anywhere in the world.
Data are gathered by simply targeting the microscopic field with the camera objective, surrounding the camera with the hands to avoid environment light interference and slowly moving the camera closer to the microscope until the microscopic field is targeted and in focus. It is then possible to take desired pictures and send them by mms with very low cost and in short time.

## Description

From any microscope and sent them by mms anywhere in the world.

This new procedure is then important not only for medlcal-diagnostic purpouses, but also for every application of microscopy in the field of industry, texiles, mining, food industry,study of matherials and plants, study and dating of archeological finds and artworks, and in any activity involving microscopy. The singularity of this system is that datas can be gatrhered with only a microscope and a cellphone and sent in real time at the cost of mms. (in the last international, tech for food forum in paris agricolture expo,the wide spread of cellphones has been underlined, since they're present in -depth in most remote villages, e.g.in guinea ,where 80% of population owns a cellphone)

Datas are gathered by just centering the microscopic field with the camera objective, surrounding this with the hand to avoid environment light interference and then moving the objective to put the field in focus. Until today the use of picture for theaching and this transmission was bound to sophisticated and expensive devices, with this method the picture can be taken and sent anywerhe in the world in real time, with a very low cost. another application of this method is theaching, in fact, the picture can be transferred directly from the phone to a computer, and be used like this or printed with no added cost.

## Claims

1. Using a simple cellphone equipped with a camera we can transmit microscopy images taken from every optical microscope to every place, drastically reducing all the costs that operation involve

2. This method was important for diagnostic procedure, for medical confirmative diagnosys in remote countries. Witch the, possibility of immediate diagnosis in every place and situation,

3. This method may be applicate to industry-texile, mining, food industry, matter technology, geology, agricultural, etc.

4. To datation of archeological finds, workarts, in evaluation and preservation of wreck

5. On every use of microscope evaluation

6. Didactic -images can be sended to a computer amd then viewed or printed.

7. For advertising, a new cellphone, a new operator, pubblicity a new server

8. This methods may be used for scientific pubblications, and for increase personal curricula

9. Can be use by governative organizations army,air force, navy, or or not governative medical humanitaries organizations.

10. This method may be used by organization as fao ,icram, to study and transmit images of foodstuff, ichtic, agricultural

11. All discipline using microscopy may have very profit by this method, for the possibility to take, use and transmit all microscopical image and send them by mms, in very short time and with very low cost.
